(19) Europäisches Patentamt
European Patent Office
Office européen des brevets

(11) **EP 0 869 766 B1**

(12) # FASCICULE DE BREVET EUROPEEN

(45) Date de publication et mention de la délivrance du brevet:
**21.11.2001 Bulletin 2001/47**

(21) Numéro de dépôt: **96939151.5**

(22) Date de dépôt: **19.11.1996**

(51) Int Cl.[7]: **A61K 7/06**

(86) Numéro de dépôt international:
**PCT/FR96/01831**

(87) Numéro de publication internationale:
**WO 97/23193 (03.07.1997 Gazette 1997/29)**

(54) **COMPOSITIONS POUR LE TRAITEMENT DES MATIERES KERATINIQUES CONTENANT L'ASSOCIATION D'UN POLYMERE POLYAMPHOLYTE ET D'UN POLYMERE CATIONIQUE**

ZUSAMMENSETZUNGEN ZUR BEHANDLUNG VON KERATINMATERIAL ENTHALTEND EIN POLYAMPHOLYT UND EIN KATIONISCHES POLYMER

COMPOSITIONS FOR THE TREATMENT OF KERATINIC MATERIALS CONTAINING THE ASSOCIATION OF A POLYAMPHOLYTE POLYMER AND A CATIONIC POLYMER

(84) Etats contractants désignés:
**DE ES FR GB IT**

(30) Priorité: **21.12.1995 FR 9515290**

(43) Date de publication de la demande:
**14.10.1998 Bulletin 1998/42**

(73) Titulaire: **L'OREAL**
**75008 Paris (FR)**

(72) Inventeurs:
- **CAUWET-MARTIN, Danièle**
  **F-75011 Paris (FR)**
- **LION, Bertrand**
  **F-93190 Livry-Gargan (FR)**
- **MONDET, Jean**
  **F-93600 Aulnay-sous-Bois (FR)**

(74) Mandataire:
**Le Blainvaux Bellegarde, Françoise et al**
**L'OREAL-DPI**
**6 rue Bertrand Sincholle**
**92585 Clichy Cedex (FR)**

(56) Documents cités:
**EP-A- 0 139 588**
**EP-A- 0 217 274**
**EP-A- 0 470 381**
**EP-A- 0 521 665**

- **International Cosmetic Ingredient Dictionary and Handbook, publié par "The Cosmetic, Toiletry and Fragance Association", Edition 2000, page 1265.**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 869 766 B1

Printed by Jouve, 75001 PARIS (FR)

## Description

La présente invention est relative à de nouvelles compositions pour le traitement des matières kératiniques, en particulier des cheveux contenant l'association d'un polymère polyampholyte et d'un polymère cationique ainsi que leurs utilisations.

On recherche depuis quelques années dans le domaine des shampooings et après-shampooings des produits conditionneurs des cheveux permettant d'apporter des effets cosmétiques supplémentaires après application et rinçage. On cherche notamment à réaliser des shampooings ou après-shampooings apportant des propriétés coiffantes.

On a envisagé dans la demande de brevet JP 7-15115 et le brevet USP 4 994 088 des compositions shampooings à base de polymères polyampholytes. Il s'agit de polymères polyélectrolytes particuliers ayant des quantités équimolaires (ou pratiquement équimolaires) de charges négatives et de charges positives. Ces polymères sont généralement insolubles dans l'eau et présentent la caractéristique de se déposer sur les cheveux par dilution et précipitation lors de l'étape de rinçage et de fixer et/ou de maintenir la chevelure après application du shampooing.

Les polymères polyampholytes présentent cependant l'inconvénient de freiner le démêlage des cheveux mouillés.

La demanderesse a découvert qu'en associant à ces polymères polyampholytes certains polymères cationiques que l'on définira plus loin, on pouvait obtenir de manière surprenante des compositions capillaires rincées apportant, après application, à la fois des propriétés coiffantes et de bonnes propriétés de démêlage des cheveux humides.

Les compositions selon l'invention sont essentiellement caractérisées par le fait qu'elles contiennent dans un milieu aqueux cosmétiquement et/ou dermatologiquement acceptable au moins :

a) un polymère polyampholyte constitué d'au moins un monomère à insaturation éthylénique et comportant dans la chaine ou latéralement à la chaîne, des quantités équimolaires ou pratiquement équimolaires de charges négatives et de charges positives ; ledit polymère étant insoluble dans l'eau à une concentration supérieure ou égale à 1% en poids et à 20° C ;

b) un polymère cationique dont la densité de charge cationique est inférieure ou égale à 4 meq/g.

Les polymères "polyampholytes" correspondant à la définition indiquée ci-dessus, conformes à l'invention présentent une charge globale proche de zéro à un pH voisin de 7.

Ces polymères sont en général insolubles dans l'eau à une concentration supérieure ou égale à 0,1% en poids et à 20° C. Certains peuvent se solubiliser dans des solutions aqueuses d'électrolytes, de préférence contenant des électrolytes minéraux. Certains peuvent également se solubiliser directement dans une base lavante à base de tensio-actifs, d'autres se solubilisent dans une base lavante en présence supplémentaire d'électrolytes. La solubilité de ces polymères dans le milieux aqueux dépendra de la structure du polyampholyte choisi. D'une façon générale, ces polymères vont pouvoir se déposer sur les cheveux par dilution et précipitation lors du rinçage, qu'ils soient appliqués en présence ou non d'électrolytes et/ou de tensioactifs.

Les polymères polyampholytes de la présente invention sont choisis, de préférence, dans le groupe constitué par :

(1) les polymères de formule suivante :

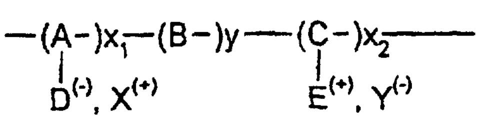

(I)

dans laquelle :

- -A- désigne un groupe résultant de la copolymérisation d'un monomère à insaturation éthylénique et portant un groupe $D^{(-)}$
- $D^{(-)}$ désigne un groupement anionique choisi dans le groupe constitué par :

(i) $-COO^{\ominus}$ ;
(ii) $-SO_3^{\ominus}$ ;
(iii) $-PO_3^{2-}$ ;

(iv) $-HPO_3^{\ominus}$ ;

- X+ désigne un cation provenant de la neutralisation des groupes D par une base minérale ou organique ;
- B- est un groupe résultant de la copolymérisation d'au moins un monomère à insaturation éthylénique, hydrophobe ou hydrophile, de préférence peu polaire ;
- C- est un groupe résultant de la copolymérisation d'un monomère à insaturation éthylénique et portant un groupement - $E^{(+)}$;
- $E^{(+)}$ désigne un groupement cationique choisi dans le groupe constitué par :

(i) 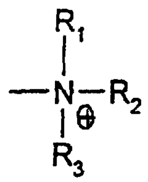

dans laquelle $R_1$, $R_2$ et $R_3$, identiques ou différents, désignent hydrogène, un groupe alkyle en $C_1$-$C_{22}$. linéaire, ramifié ou cyclique (cycloaliphatique ou aromatique) ;

(ii) 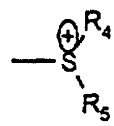

dans laquelle $R_4$ et $R_5$, identiques ou différents, désignent un groupe aliphatique, cycloaliphatique ou aromatique ;

(iii) 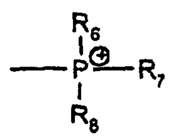

où $R_6$, $R_7$ et $R_8$ identiques ou différents, désignent un groupe aliphatique, cycloaliphatique ou aromatique.
- $Y^{(-)}$ désigne un anion résultant de la neutralisation des groupes E par un acide minéral ou organique ou de la quatemisation des groupes E ;
$x_1$, $x_2$ et y désignent respectivement les pourcentages en moles en groupe A, en groupe B et en groupe C ; $x_1$ et $x_2$ étant identiques ou pratiquement identiques de telle sorte que la charge globale du polymère soit proche de 0 pour un pH voisin de 7 ; la somme $x_1 + x_2$ étant de préférence supérieure ou égale à 40 % mole et y étant de préférence inférieur ou égale à 60 % mole.

(2) les polymères bétaïniques ayant pour monomère de base celui de formule suivante :

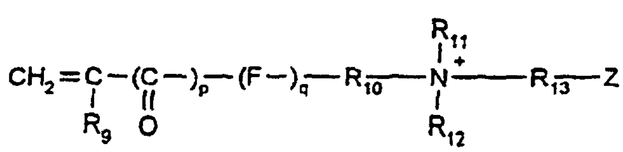 (II).

dans laquelle:

$R_9$, $R_{11}$ et $R_{12}$ désignent, identiques ou différents un hydrogène ou un alkyle en $C_1$-$C_4$, linéaire ou ramifié ;
Z désigne $COO^{(-)}$, $SO_3^{(-)}$ ou $HPO_3^{(-)}$ ;
F désigne -NH ou O ou forme avec le groupe $R_{10}$ un cycle ou hétérocycle, aromatique ou non-aromatique en $C_5$-$C_7$;
$R_{10}$ et $R_{13}$ désignent, indépendamment l'un de l'autre, un groupe hydrocarboné divalent en particulier un

groupe $-(CH_2)_n-$ avec n entier allant de 1 à 4 ;
$R_{10}$ peut former avec $R_{11}$ et $R_{12}$ un hétérocycle en $C_5$-$C_7$ ;
p vaut 0 ou 1 et q vaut 0 ou 1.

Dans la formule générale (I) telle que définie ci-dessus ; les monomères conduisant après copolymérisation aux motifs de structure :

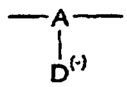

- lorsque $D^{(-)}$ désigne la fonction carboxylate, sont choisis parmi les sels d'acides carboxyliques linéaires, ramifiés ou cycliques (cycloaliphatiques ou aromatiques tels que les sels de l'acide crotonique, de l'acide acrylique, de l'acide méthacrylique, de l'acide vinylbenzoïque ; les sels des diacides carboxyliques tels que les sels de l'acide maléique, fumarique ou itaconique ainsi que leurs monoesters et monoamides ;

- lorsque $D^{(-)}$ désigne la fonction sulfonate, sont choisis parmi les sels de l'acide acrylamido 2-méthyl-2 propane sulfonique, de l'acide vinylsulfonique et de l'acide styrène sulfonique sous forme neutralisée ; les sels du (méth) acrylate de 2-sulfoéthyle ;

- lorsque $D^{(-)}$ désigne la fonction phosphonate, sont par exemple les sels de l'acide vinylphosphonique neutralisé.

Le contre ion $X^{(+)}$ associé à $D^{(-)}$ résulte en général de la neutralisation du groupe D par une base minérale telle que NaOH ou KOH ou une base organique telle qu'une amine ou un aminoalcoot.
Dans la formule générale (I), les monomères conduisant aux motifs de structure -B-sont choisis parmi les monomères à insaturation éthylénique hydrophiles ou hydrophobes, plus particulièrement peu polaires et sont choisis de telle sorte que le polymère final ne soit pas soluble dans l'eau en l'absence d'électrolyte et/ou de tensio-actif.
A titre d'exemple, on peut citer les monomères vinyliques tels que les esters vinyliques en $C_1$-$C_{24}$, linéaires, ramifiés ou cycliques, les oléfines comme l'éthylène, le styrène et ses dérivés substitués ; les esters ou les amides d'acide (méth)acrylique en $C_1$-$C_{24}$ linéaires, ramifiés ou cycliques.
Les monomères conduisant par copolymérisation aux motifs -B- peuvent être copolymérisés avec des macromères siliconés présentant une fonction vinylique terminale. Ils peuvent également être copolymérisés par des monomères à groupes fluorés ou perfluorés du type vinylique, allylique ou (méth)acrylique comme par exemple le fluorure de vinylidène, le chlorotrifluoroéthylène, le tétrafluoroéthylène ; les (meth)acrylates perfluorés comme le (méth)acrylate de perfluorohexyle ou de perfluorooctyle.
Dans la formule générale (I), les monomères conduisant après copolymérisation aux motifs de structure :

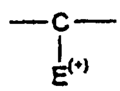

sont choisis, de préférence parmi les monomères, du type (méth)acrylique, vinylique, allylique ou diallylique comportant une fonction amine tertiaire quatemisée par un halogénure d'alkyle ou un sulfate de dialkyle.
On peut citer par exemple :

- le diméthylaminoéthyl(méth)acrylate,
- le diéthylaminoéthyl(méth)acrylate,
- le diméthylaminopropyl(méth)acrylate,
- le diméthylaminopropyl(méth)acrylamide,
- le 2-vinylpyridine,
- la 4-vinylpyridine,
- la diméthylallylamine,

quatemisés par un halogénure d'alkyle ou un sulfate de dialkyle.
Les polyampholytes de formule (I) particulièrement préférés sont choisis parmi les copolymères styrène sulfonate de sodium / chlorure de triméthylammonio éthylméthacrylate et les copolymères styrène sulfonate / chlorure de triméthylammonio propyl-(méth)acrylamide.
Les poids moléculaires des polyampholytes peuvent varier de 500 à 50.000.000 et sont de préférence supérieurs à 10.000.

Les polyampholytes de formule (II) particulièrement préférés sont choisis dans le groupe constitué par :

- le poly 1-vinyl-2-(3-sulfopropyl) imidazolium hydroxyde (J.C. SALAMONE, Polymer 1978, vol 19, P 1157);
- le poly 1-vinyl-3-(3-sulfopropyl) imidazolium hydroxyde ;
- le poly 1-vinyl-3-(4-sulfobutyl) imidazolium hydroxyde ;
- le poly 1-vinyl-2-méthyl-3-(4-sulfobutyl) imidazolium hydroxyde ;
- le poly 2-vinyl-1-(3-sulfopropyl) pyridinium hydroxyde ;
- le poly 2-méthyl-5-vinyl-1-(3-sulfopropyl) pyridinium hydroxyde ;
- le poly 4-vinyl-1-(3-sulfopropyl) pyridinium hydroxyde ;
- le poly diméthyl (2 méthacryloxyéthyl) (3 sulfopropyl) ammonium hydroxyde ;
- le polydiéthyl (2-méthacryloxyéthoxy-2-éthyl)(3-sulfopropyl) ammonium hydroxyde ;
- le poly 4-vinyl-4-(sulfobutyl)pyridinium hydroxyde ;
- le poly N-(3-sulfopropyl) N méthacrylamidopropyl N, N-diméthyl ammonium bétaïne.

Ces polysulfobétaïnes sont citées dans Encyclopedia of Polymer Science and Engineering - Second Edition - vol 11, p 517.

Les polyampholytes de formule (f) peuvent être synthétisés par copolymérisation directe en solution dans l'eau avec ou sans électrolyte, par polymérisation en solution en milieu eau/solvant organique. Ils peuvent être obtenus par copolymérisation par précipitation ou polymérisation en dispersion dans un milieu eau/solvant organique.

Les méthodes générales de polymérisation de ces polymères sont décrites dans l'Encyclopedia of Polymer Science and Engineering, Second Edition, vol 11, p 521, Wiley Interscience.

Ils peuvent également être obtenus par polymérisation en émulsion inverse en présence d'eau et d'un solvant organique selon le procédé décrit dans l'article de J. M. CORPART et F. CANDAU, Macromolecules, vol. 26, n° 6 p 1333, 1993.

Ils peuvent être obtenus aussi par copolymérisation «miscellaire» dans l'eau suivant un procédé décrit dans l'article I. LACIK, Polymer, (1995), 36 (16), 3197-3211.

Lorsque la polymérisation est effectuée en solution dans l'eau, on utilise de préférence un amorceur hydrosoluble comme le persulfate de sodium ou de potassium ou un système redox.

Lorsque la polymérisation est effectuée en milieu organique ou hydroorganique, on peut également utiliser des amorceurs organiques. Pour contrôler le poids moléculaire final, la présence d'un agent de transfert peut être nécessaire dans le milieu de polymérisation.

Les monomères constituant les polyampholytes de l'invention sont de préférence déjà neutralisés et/ou quatemisés. Lorsqu'on opère avec des monomères déjà neutralisés en solution dans l'eau, on observe, lors de la polymérisation, une autoneutralisation des groupes anioniques et cationiques entre eux ; ce qui peut provoquer une précipitation du polymère formé.

Lorqu'on opère en milieu dilué dans l'eau, on observe, lors de la polymérisation, une expulsion totale ou partielle des contre ions $X^+$ et $Y^-$ dans la solution aqueuse.

Les polyampholytes de formule (I) peuvent également être obtenus par polymérisation directe de la paire d'ions monomères :

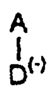 et 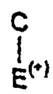

selon la méthode décrite par J.C. SALAMONE dans l'article extrait du J. Macromol. Sc. Chem. A22 (5-7) p 653-664 (1985).

Les polymères polyampholytes de formule (II) peuvent être obtenus par synthèse du monomère déjà bétaïnisé en effectuant une réaction de quaternisation puis une polymérisation. Ils peuvent également être obtenus par réalisation du polymère à groupes amines puis par quatemisation.

Les polymères polyampholytes de l'invention sont présents dans les compositions de l'invention dans des proportions allant de préférence de 0,01 à 20 % en poids et plus particulièrement de 0,1 à 10 % en poids par rapport au poids total de la composition.

Les polymères cationiques utilisés conformément à l'invention ont en général un poids moléculaire moyen en masse d'au moins 5000, préférentiellement d'au moins 10.000 et inférieur à 10.000.000 et plus particulièrement allant de 100.000 à 2.000.000. Ils ont en général des motifs contenant un atome d'azote tels que des motifs ammoniums quaternaires ou amino ou leurs mélanges. Leur densité de charge cationique est inférieure ou égale à 4 meq/g et de

préférence supérieure ou égale à 0,9 meq/g et plus préférentiellement comprise entre 1,1 et 3 meq/g. La densité de charge peut être déterminée selon la méthode Kjeldahl. Elle correspond en général à un pH de l'ordre de 3 à 9.

Parmi les polymères cationiques utilisables selon l'invention, on peut citer les copolymères de monomères vinyliques ayant des fonctions amines ou ammoniums quaternaires avec des monomères à insaturation éthylénique hydrosolubles tels que l'acrylamide, le méthacrylamide, les alkyl- ou dialkyl(méth)acrylamides, les alkyl(méth)-acrylates, le vinylcaprolactone, le vinylpyrrolidone ; ou biens d'autres monomères tels que les esters vinyliques, l'alcool vinylique, l'anhydride maléique, le propylène-glycol, l'éthylène glycol. Les groupes alkyls ou dialkyls des fonctions amines ou ammoniums sont de préférence en $C_1$-$C_9$ et plus préférentiellement en $C_1$-$C_3$.

Les amines peuvent être primaires, secondaires ou tertiaires. Les amines secondaires et tertiaires sont préférentielles.

Les monomères vinyliques amino-substitués peuvent être polymérisés sous leur forme amine puis éventuellement quatemisés. Les amines peuvent être également quatemisées après la formation du polymère. Par exemple, les fonctions amines tertiaires peuvent être quaternisées par réaction avec un sel de formule R'X où R' est un radical alkyle de chaîne courte (en $C_1$-$C_7$ de préférence et plus particulièrement en $C_1$-$C_3$) et X est un anion formant un sel hydrosoluble avec l'ammonium quaternaire.

Parmi les monomères vinyliques à fonction amines ou ammoniums quaternaires, on peut citer par exemple des composés vinyliques substitués par un groupe du type dialkylaminoalkyl (méth)acrylate, monoalkyl-aminoalkyl (méth)acrylate ; des sels de trialkyl-méthacryloxyalkyl ammonium ; des sels diallyliques d'ammonium quaternaire ; des monomères vinyliques quaternaires ayant des cycles portant des atomes d'azote tels que pyridinium, imidazolium, pyrrolidone quaternisée comme alkylvinylimidazolium, alkyl-vinylpyridinium, les sels quaternaires d'alkylvinyl pyrrolidone. Les portions alkyls de ces monomères sont de préférence des alkyls en $C_1$-$C_3$ et plus préférentiellement des alkyls en $C_1$ ou $C_2$.

On peut également citer comme monomères vinyliques amino-substitués, les dialkylaminoalkyl (méth)acrylates, les dialkylaminoalkyl (méth)acrylamides. Les groupes alkyls ou dialkyls sont de préférence en $C_1$-$C_9$ et plus préférentiellement en $C_1$-$C_3$.

Les polymères cationiques de l'invention peuvent comprendre des mélanges de monomères vinyliques dérivés d'amines et/ou de monomères vinyliques dérivés d'ammoniums quaternaires et/ou d'autres monomères compatibles. On peut citer, à titre d'exemple :

- les copolymères de 1-vinylpyrrolidone et de sel de 1-vinyl-3-méthylimidazolium (chlorure par exemple) (appelé Polyquatemium-16 dans le CTFA) tels que ceux vendus sous le nom LUVIQUAT par la société BASF ;

- les copolymères de 1-vinyl-2-pyrrolidone et de diméthylaminoéthylméthacrylate (appelés Polyquatemium-11 selon le CTFA) tels que ceux vendus sous le nom GAFQUAT (par exemple le GAFQUAT 755N) par la société GAF CORPORATION ;

- les homopolymères de chlorure de diméthyl-diallylammonium (Polyquatemium 5 selon le CTFA) et les copolymères d'acrylamide et de chlorure de diméthyl-diallylammonium (Polyquatemium-7 selon le CTFA) tels que ceux vendus sous le nom MERQUAT 550 et MERQUAT S par la Société MERCK ;

- les sels d'acides minéraux d'aminoalkylesters d'homo et de copolymères d'acides carboxyliques insaturés ayant de 3 à 5 atomes de carbone tels que ceux décrits dans le brevet USP 4 009 256.

Parmi les polymères cationiques utilisables, on peut citer aussi les polysaccharides cationiques tels que les dérivés de cellulose cationiques et les dérivés de l'amidon cationiques.

Parmi les polysaccharides cationiques, on peut citer les polymères de formule :

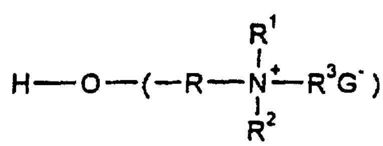

où :

H est un reste d'anhydroglucose tel que l'amidon ou un reste d'anhydroglucose cellulosique ;
R est un alkylène, un oxyalkylène, un polyoxyalkylène ou un hydroxyalkylène ou leurs mélanges ;

$R^1$, $R^2$ et $R^3$, identiques ou différents, désignent un groupe alkyle, aryle, alkylaryle, arylalkyle, alkoxyalkyle ou alkoxyaryle ; chaque groupe contenant jusqu'à 18 atomes de carbone et le nombre total d'atomes de carbone par unité cationique est de préférence inférieure ou égale à 20.
$G^-$ est un anion résultant de la quaternisation de l'amine $NR^1R^2R^3$.

Parmi les polymères cellulosiques cationiques, on peut citer ceux vendus par la société AMERCHOL CORP. sous les noms JR et LR tels que les sels d'hydroxyéthylcellulose quaternaires obtenus par réaction avec un époxyde susbtitué par un triméthylammonium (polyquaternium-10 selon le CTFA). On peut citer également les sels d'hydroxyéthyl-cellulose quaternaires obtenus par réaction avec un époxyde substitué par le lauryl-diméthylammonium (polyquatemium-24 selon le CTFA) comme ceux vendus sous le nom POLYMER LM200 par AMERCHOL CORP.
On peut citer également, comme polymères cationiques utilisables selon l'invention, les dérivés de gomme de guar cationiques tels que le chlorure d'hydroxypropyltrimonium de guar vendu sous les noms JAGUAR par la société CELANESE CORP.
On peut citer également les éthers celluloses quaternaires telles que celles décrites dans le brevet USP. 3 962 418 et les copolymères de cellulose étherifiés et d'amidon tels que ceux décrits dans le brevet US 3 958 581.
Les polymères cationiques de l'invention sont présents dans les compositions dans des proportions allant, de préférence, de 0,01 à 5 % en poids et de préférence de 0,1 à 3 % en poids par rapport au poids total de la composition.
Les compositions aqueuses de l'invention peuvent contenir en plus des électrolytes minéraux ou organiques permettant de solubiliser les polymères polyampholytes.
Les électrolytes utilisés sont de préférence des sels hydrosolubles minéraux tels que les sels de métaux alcalins, les sels de métaux alcalino-terreux ou les sels d'aluminium d'acide chlorhydrique, sulfurique ou nitrique ou d'acide organique comme l'acide citrique, lactique ou tartrique. Les électrolytes particulièrement préférés sont choisis parmi le sulfate de potassium, le sulfate de sodium, le sulfate de magnésium, le nitrate de calcium, le nitrate d'aluminium, le nitrate de magnésium, le chlorure de sodium, ie chlorure de potassium, le chlorure d'aluminium, le carbonate de potassium, le carbonate de sodium, le carbonate d'aluminium, le citrate de sodium.
Ils sont présents dans des proportions allant de 0,1 à 30 % en poids et plus préférentiellement de 1 à 10 % en poids par rapport au poids total de la composition.
Le pH des compositions aqueuses conformes à l'invention est ajusté de préférence entre 3 et 11 et plus particulièrement entre 5 et 9 par l'emploi d'agents alcalinisants ou acidifiants ou de tampons. Il est important d'ajuster le pH de telle sorte que le polymère polyampholyte sous forme dissoute ou dispersée dans le milieu aqueux de la composition, puisse précipiter par dilution lors de l'étape de rinçage après application de la composition sur les matières kératiniques.
Les compositions selon l'invention, lorsqu'elles se présentent en particulier sous forme de shampooing comprennent une base lavante, généralement aqueuse. Cette base de tensioactifs peut également servir à solubiliser dans le milieu aqueux le ou les polyampholytes.
Le ou les tensioactifs formant la base lavante peuvent être indifféremment choisis, seuls ou en mélanges, au sein des tensioactifs anioniques, amphotères, non ioniques, zwittérioniques et cationiques.
La quantité minimale de base lavante est celle juste suffisante pour conférer à la composition finale un pouvoir moussant et/ou détergent satisfaisant et/ou pour solubiliser les polyampholytes présents dans la composition.
Ainsi, selon l'invention, la base lavante peut représenter de 4 % à 30 % en poids, de préférence de 10 % à 25 % en poids, et encore plus préférentiellement de 12 % à 20 % en poids, du poids total de la composition finale.
Les tensioactifs convenant à la mise en oeuvre de la présente invention sont notamment les suivants :

(i) <u>Tensioactif(s) anionique(s)</u> :

Leur nature ne revêt pas, dans le cadre de la présente invention, de caractère véritablement critique.
Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, $\alpha$-oléfine-sulfonates, paraffine-sulfonates ; les alkylsulfosuccinates, les alkytéthersulfo-succinates, les alkylamidesuffosuccinates ; les alkyl-sulfosuccinamates ; les alkylsulfo-acétates ; les alkylétherphosphates ; les acylsarcosinates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyllactylates dont le radical acyle comporte 8 à 20 atomes de carbone. On peut également utiliser des tensioactifs faiblement anioniques, comme les acides d'alkyl D

galactoside uroniques et leurs sels ainsi que les acides éthers carboxyliques polyoxyafkylénés et leurs sels, en particulier ceux comportant de 2 à 50 groupements oxyde d'éthylène, et leurs mélanges. Les tensioactifs anioniques du type acides ou sels d'éthers carboxyliques polyoxyalkylénés sont en particulier ceux qui répondent à la formule (I) suivante :

$$R_1—(-OC_2H_4-)_n—OCH_2COOA \quad (1)$$

dans laquelle :

> $R_1$ désigne un groupement alkyle ou alkylaryle, et n est un nombre entier ou décimal (valeur moyenne) pouvant varier de 2 à 24 et de préférence de 3 à 10, le radical alkyle ayant entre 6 et 20 atomes de carbone environ, et aryle désignant de préférence phényle,
>
> A désigne H, ammonium, Na, K, Li, Mg ou un reste monoéthanolamine ou triéthanolamine. On peut également utiliser des mélanges de composés de formule (1) en particulier des mélanges dans lesquels les groupements $R_1$ sont différents.

Des composés de formule (1) sont vendus par exemple par la Société CHEM Y sous les dénominations AKYPOS (NP40, NP70, OP40, OP80, RLM25, RLM38, RLMQ38 NV, RLM 45, RLM 45 NV, RLM 100, RLM 100 NV, RO20, RO 90, RCS 60, RS 60, RS 100, RO 50) ou par la Société SANDOZ sous les dénominations SANDOPAN (DTC Acid, DTC).

(ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthytène ; les esters d'acides gras du sorbitan oxyéthylénés ayant de 2 à 30 moles d'oxyde d'éthylène ; les esters d'acides gras du sucrose, les esters d'acides gras de polyéthylèneglycol, les alkylpolyglycosides, les dérivés de N-alkyl glucamine, les oxydes d'amines tels que les oxydes d'alkyl ($C_{10}$-$C_{14}$) amines ou les oxydes de N-acylaminopropylmorpholine. On notera que les alkylpolyglycosides constituent des tensioactifs non-ioniques rentrant particulièrement bien dans le cadre de la présente invention.

(iii) Tensioactif(s) amphotère(s) ou zwittérionique(s) :

Les agents tensioactifs amphotères ou zwitterioniques, dont la nature ne revet pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) betaïnes ou les alkyl ($C_8$-$C_{20}$) amidoalkyl ($C_1$-$C_6$) sulfobétaïnes.

Parmi les dérivés d'amines, on peut citer les produits vendus sous la dénomination MIRANOL, tels que décrits dans les brevets US-2528378 et US-2781354 et classés dans le dictionnaire CTFA, 3ème édition, 1982, sous les dénominations Amphocarboxy-glycinates et Amphocarboxypropionates de structures respectives :

$$R_2\text{-CONHCH}_2\text{CH}_2\text{-N}(R_3)(R_4)(\text{CH}_2\text{COO-}) \quad (2)$$

dans laquelle : $R_2$ désigne un radical alkyle d'un acide $R_2$-COOH présent dans l'huile de coprah hydrolysée, un radical

heptyle, nonyle ou undécyle, $R_3$ désigne un groupement bêta-hydroxyéthyle et $R_4$ un groupement carboxyméthyle ; et

$$R_2\text{-CONHCH}_2\text{CH}_2\text{-N(B)(C)} \qquad (3)$$

dans laquelle:

B représente $-CH_2CH_2OX'$, C représente $-(CH_2)_z$ -Y', avec z = 1 ou 2,
X' désigne le groupement $-CH_2CH_2-COOH$ ou un atome d'hydrogène
Y' désigne -COOH ou le radical $-CH_2$ - CHOH - $SO_3H$
$R_2$. désigne un radical alkyle d'un acide $R_9$ -COOH présent dans l'huile de coprah ou dans l'huile de lin hydrolysée, un radical alkyle, notamment en $C_7$, $C_9$, $C_{11}$ ou $C_{13}$, un radical alkyte en $C_{17}$ et sa forme iso, un radical $C_{17}$ insaturé.

A titre d'exemple on peut citer le cocoamphocarboxyglycinate vendu sous la dénomination commerciale MIRANOL $C_2M$ concentré par la Société MIRANOL.

(iv) Tensioactifs cationiques :

Parmi les tensioactifs cationiques, dont la nature ne revet pas dans le cadre de la présente invention de caractère critique, on peut citer en particulier (liste non limitative) : les sels d'amines grasses primaires, secondaires ou tertiaires, éventuellement polyoxy-alkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.
On notera que les tensioactifs cationiques, dont l'utilisation n'est pas exclue, ne constituent pas des tensioactifs préférés pour la mise en oeuvre de la présente invention.
Le milieu cosmétiquement ou dermatologiquement acceptable des compositions de l'invention est de préférence de l'eau ou une solution hydroalcoolique d'un alcool inférieur tels que l'éthanol, l'isopropanol ou le butanol.
Les compositions selon l'invention peuvent bien entendu contenir en outre des adjuvants usuels dans le domaine des compositions capillaires comme par exemple des parfums, des conservateurs, des séquestrants, des épaississants, des adoucissants, des modificateurs de mousse, des colorants, des agents nacrants, des agents hydratants, des agents anti-pelliculaires ou antiséborrhéiques, des vitamines, des filtres solaires et autres.
Bien entendu, l'homme de l'art veillera à choisir ce ou ces éventuels composés complémentaires et/ou leurs quantités de manière telle que les propriétés avantageuses attachées intrinsèquement à la composition conforme à l'invention ne soient pas, ou substantiellement pas, altérées par la ou les adjonctions envisagées.
Ces compositions peuvent se présenter sous la forme de liquides plus ou moins épaissis, de crèmes ou de gels et elles conviennent principalement au lavage, au soin et/ou le coiffage des cheveux. Elles peuvent aussi se présenter sous la forme de lotions à rincer.
Un autre objet de l'invention consiste en un procédé de traitement non-thérapeutique des cheveux caractérisé par le fait qu'on applique directement sur les cheveux mouillés une composition telle que définie ci-dessus, et après un éventuel temps de pause, qu'on effectue un rinçage à l'eau ; ledit procédé pouvant être répété plusieurs fois.
Comme indiqué précédemment, les compositions selon l'invention confèrent aux cheveux, après rinçage, un remarquable effet coiffant qui se manifeste notamment par une facilité de coiffage et de maintien ainsi qu'un démêlage des cheveux mouillés amélioré sensiblement.
Les exemples qui suivent servent à illustrer la présente invention sans toutefois présenter un caractère limitatif.

**EXEMPLES**

EXEMPLE DE PREPARATION 1

*Synthèse du copolymère de styrène sulfonate de sodium et de chlorure de triméthylammonio éthyl-méthacrylate (50/50 % mole) de formule (I).*

Dans un réacteur avec agitation mécanique centrale, réfrigérant, thermomètre et barbotage d'azote, on introduit 49,8 g de styrène sulfonate de sodium en poudre. On introduit ensuite 63,63 g d'une solution aqueuse à 78,9 % d'extrait sec en chlorure de triméthylammonio éthyl-méthacrylate. On introduit ensuite 300 g d'eau permutée plus 2 g de persulfate de potassium (amorceur de polymérisation). Tous ces ajouts sont faits à température ambiante.

On agite le milieu réactionnel à 400 tours/min pour obtenir une dissolution et une homogénéisation.
On effectue un barbotage d'azote. On chauffe le milieu à 72° C et on maintient cette température, sous agitation pendant 24 heures.
En fin de polymérisation, le milieu réactionnel est ramené à température ambiante. Le milieu est trouble mais le polymère ne précipite pas.
On purifie le polymère par précipitation de la solution de synthèse dans 5 l d'eau permutée. On récupère le précipité. On sèche en étuve sous vide et à 45° C jusqu'à obtention d'un poids constant.
L'analyse élémentaire est conforme à celle du polymère théorique. Le rendement obtenu est de 85 %.
Le polymère ainsi obtenu est insoluble dans l'eau à 1 % en poids.
Il peut se solubiliser dans l'eau à une concentration de 1 % par l'ajout d'une quantité minimale de 3,85 % en poids de NaCl.
Il peut également se solubiliser dans une base lavante constituée de :

- lauryl éther sulfate de sodium 15 % en poids
- cocoylbétaïne 3 % en poids
- eau qsp 100 % en poids

à la concentration de 1 % en poids par l'ajout d'une quantité minimale de 2 % de NaCl.

<u>EXEMPLE DE PREPARATION 2</u>

*Synthèse du copolymère de styrène sulfonate de sodium et de chlorure de triméthylammonio propyl-méthacrylamide (50/50 % mole).*

On opère dans les mêmes conditions que celles mises en oeuvre dans l'exemple 1 en utilisant 48,3 g de styrène sulfonate de sodium, 98,48 g d'une solution aqueuse à 52,5 % d'extrait sec de chlorure de triméthylammonio propylméthacrylamide, 500 g d'eau permutée et de 2 g de persulfate d'ammonium.
L'analyse élémentaire est conforme à celle du polymère théorique. Le rendement obtenu est de 91 %.
Le polymère ainsi obtenu est insoluble dans l'eau à une concentration de 1 % en poids. Ils peut se solubiliser dans l'eau à cette même concentration en présence d'au moins 5,66 % du poids de NaCl.
Il peut se solubiliser dans la base lavante de l'exemple 1 à cette même concentration en présence d'au moins 4,76 % en poids de NaCl.

<u>EXEMPLE DE PREPARATION 3</u>

*Synthèse du copolymère de l'acide acrylamido-2-méthyl-2-propane sulfonique et de chlorure de triméthyl ammonio éthyl-méthacrylate (50/50 % mole)*

On opère dans le même réacteur que celui mis en oeuvre dans l'exemple 1 en utilisant 47,42 g d'acide acrylamido-2-méthyl-2-propane sulfonique, 60,25 g d'une solution aqueuse à 78,9 % d'extrait sec de chlorure de triméthylammonio-éthyl méthacrylate.
Avant l'introduction du monomère quaternaire, on introduit dans le réacteur le monomère suifonique puis 200 g d'eau permutée pour une dissolution et homogénéisation à température ambiante.
On neutralise le monomère sulfonique par ajout de 26,3 g de NaOH à 35 %, sous agitation à température ambiante. On introduit ensuite le monomère quaternaire et on ajoute 150 g d'eau permutée et 2 g de persulfate d'ammonium. On agite, on effectue un barbotage à l'azote et on chauffe à 72° C pendant 24 heures. On obtient une solution trouble. Cette solution concentrée précipite dans l'eau mais le précipité est récupéré et purifié par précipitation de la solution de synthèse dans 5 l d'éthanol. On sèche en étuve jusqu'à obtention d'un poids constant. Le rendement obtenu est de 85 %.
Le polymère obtenu se dissout directement à une concentration de 1 % en poids dans la base lavante de l'exemple 1. Au delà de 4 % en poids dans cette base lavante, il faut rajouter du NaCl pour maintenir la solubilisation.

<u>EXEMPLE DE PREPARATION 4</u>

*Synthèse du copolymère du sel de sodium de l'acide acrylamido-2-méthyl-2-propane sulfonique et du chlorure de triméthylammonio propyl-méthacrylamide (50/50 % mol)*

On réalise une solution aqueuse à 49,15 % d'extrait sec en sel de sodium de l'acide acrylamido-2-méthyl-2-propane sulfonique par neutralisation de l'acide par une quantité stoechiométrique de NaOH.

Dans un réacteur identique à celui de l'exemple 1, on introduit 103,68 g de ladite solution aqueuse puis 93,43 g d'une solution aqueuse à 52,5 % d'extrait sec de chlorure de triméthylammonio propyl-méthacrylamide. On ajoute ensuite 300 g d'eau permutée et 2 g de persulfate d'ammonium.

On procède ensuite dans les mêmes conditions que celles mises en oeuvre dans l'exemple 3. Le rendement obtenu est de 88 %.

Le polymère ainsi obtenu est insoluble dans l'eau à une concentration à 1 % et se solubilise à la même concentration en présence d'au moins 3,62 % de NaCl. Il se dissout directement à la même concentration dans la base lavante de l'exemple 1. Au delà de 5,5 % en poids, il faut ajouter un électrolyte pour maintenir sa dissolution.

| **Exemple A : Shampooing** | |
|---|---|
| Lauryl éther sulfate de sodium à 22 moles d'oxyde d'éthylène vendu par la Société ALBRIGHT et WILSON sous le nom | |
| d'EMPICOL ESB B/FL | 24 g MA |
| Cocoylbétaïne en solution aqueuse à 32 % | 8 g MA |
| Polymère de l'exemple 1 | 1 g MA |
| NaCl | 2g |
| Copolymère chlorure de dimethyldiallylammonium/ acrylamide 50/50 en solution aqueuse à 8 % | 1 g MA |
| Conservateurs, parfums | |
| Eau qsp | 100 g |

pH ajusté à 7 par HCl.

Ce shampooing possède une mousse douce et onctueuse et confère à la chevelure une bonne discipline et une bonne tenue.

| **Exemple B : Lotion rincée** | |
|---|---|
| Copolymère chlorure de méthacrylate de triméthyl éthyl ammonium/ acrylamide (42/58) véhiculé en dispersion à 50 % dans l'huile | 0,3 g MA |
| Polymère de l'exemple 1 | 2 g MA |
| NaCl | 3 g |
| Parfums, colorants, sequestrants | |
| Eau qsp | 100 g |

Cette lotion s'applique facilement sur les cheveux. Après rinçage, les cheveux sont faciles à démêler et à coiffer.

| **Exemple C : Après-shampooing** | |
|---|---|
| Chlorure de cétyltriméthylammonium en solution aqueuse à 29 % vendu par LONZA sous le nom de BURQUAT CT29 | 1 g MA |
| Chlorure de béhényl triméthyl ammonium à 80 % dans un mélange eau/isopropanol (15/81) | 2 g MA |
| Hydroxyéthylcellulose réticulée à l'épichlorhydrine quatemisé par triméthylamine | 0,5 g MA |
| Mélange alcool cétylstéarylique et cétylstéarylique oxyéthylène 33 moles d'oxyde d'éthylène (88/20) | 2 g |
| Gomme de xanthane | 0,5 g |
| NaCl | 3 g |
| Polymère de l'exemple 1 | 2 g |
| Eau qsp | 100 g |

pH ajusté à 7 avec NaOH.

Cet après-shampooing, après rinçage, apporte tenue et douceur aux cheveux.

## Revendications

**1.** Composition pour le traitement des matières kératiniques, **caractérisée par le fait qu'**elle contient dans un milieu aqueux cosmétiquement et/ou dermatologiquement acceptable au moins:

a) un polymère polyampholyte constitué d'au moins un monomère à insaturation éthylénique et comportant dans la chaine ou latéralement à la chaîne, des quantités équimolaires ou pratiquement équimolaires de charges négatives et de charges positives ; ledit polymère étant insoluble dans l'eau à une concentration supérieure ou égale à 1 % en poids et à 20° C ;
b) un polymère cationique dont la densité de charge cationique est inférieure ou égale à 4 meq/g.

**2.** Composition selon la revendication 1, **caractérisée par le fait que** le polymère polyampholyte est choisi parmi les polymères répondant à la formule suivante:

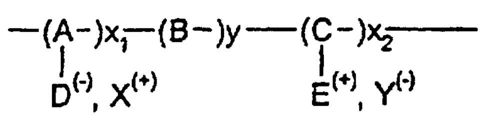

(I)

dans laquelle:

- A- désigne un groupe résultant de la copolymérisation d'au moins un monomère à insaturation éthylénique et portant un groupe $D^{(-)}$ ;
- $D^{(-)}$ désigne un groupe anionique choisi dans le groupe constitué par :

  (i) $—COO^{\ominus}$ ;
  (ii) $—SO_3^{\ominus}$ ;
  (iii) $—PO_3^{2-}$ ;
  (iv) $—HPO_3^{\ominus}$ ;

- X+ désigne un cation provenant de la neutralisation des groupes D par une base minérale ou organique ;
- B- désigne un groupe résultant de la copolymérisation d'au moins un monomère à insaturation éthylénique hydrophile ou hydrophobe ;
- C- est un groupe résultant de la copolymérisation d'au moins un monomère à insaturation éthylénique et portant un groupe -E(+) ;
- E(+) désigne un groupement cationique choisi dans le groupe constitué par:

(i) 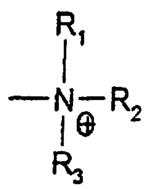

dans laquelle $R_1$, $R_2$ et $R_3$, identiques ou différents, désignent hydrogène, un groupe alkyle en $C_1$-$C_{22}$, linéaire, ramifié ou cyclique (cycloaliphatique ou aromatique) ;

(ii) 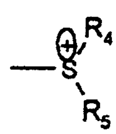

dans laquelle $R_4$ et $R_5$, identiques ou différents, désignent un groupe aliphatique, cycloaliphatique ou aromatique ;

(iii) 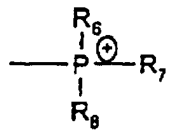

où $R_6$, $R_7$ et $R_8$ identiques ou différents, désignent un groupe aliphatique, cycloaliphatique ou aromatique.

- $Y^{(-)}$ désigne un anion résultant de la neutralisation des groupes E par un acide minéral ou organique ou par quatemisation des groupes E ;

$x_1$, $x_2$, y désignent respectivement la concentration en mole du groupe A, du groupe B et du groupe C $x_1$ et $x_2$ étant identiques ou pratiquement identiques de telle sorte que la charge globale du polymère soit proche de zéro à un pH voisin de 7.

**3.** Composition selon la revendication 2, **caractérisée par le fait que** dans la formule (I), la somme $x_1$ + $x_2$ est supérieure ou égale à 40 % mol et y est inférieur ou égal à 60 % mol.

**4.** Composition selon la revendication 2 ou 3, **caractérisée par le fait que** dans la formule (I), D(-) désigne carboxylate et

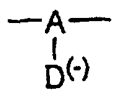

est choisi dans le groupe constitué par
les sels des acides carboxyliques, linéaires, ramifiés ou cycliques ; les sels des diacides carboxyliques linéaires, ramifiés ou cycliques et leurs monoesters ou monoamides.

**5.** Composition selon la revendication 2 ou 3, **caractérisée par le fait que** dans la formule
(I), $D^{(-)}$ désigne sulfonate et

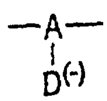

est choisi dans le groupe constitué par les sels de l'acide acrylamido-2-méthyl-2-propane sulfonique, de l'acide vinylsulfonique, de l'acide styrène sulfonique, les sels du méthacrylate de 2-sulfoethyle.

**6.** Composition selon la revendication 2 ou 3, **caractérisée par le fait que** $D^{(-)}$ désigne phosphonate et

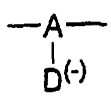

désigne un sel de l'acide vinyl phosphonique.

**7.** Composition selon l'une quelconque des revendications 2 à 6, **caractérisée par le fait que** le groupe -B- est constitué par au moins un monomère choisi parmi les esters vinyliques en $C_1$-$C_{24}$, linéaires, ramifiés ou cycliques, les oléfines, le styrène et ses dérivés substitués, les esters ou les amides de l'acide (meth)acrylique en $C_1$-$C_{24}$, linéaires ramifiés ou cycliques et éventuellement par au moins un monomére choisi dans le groupe constitué par des macroméres siliconés présentant une fonction vinylique terminale, des monomères vinyliques, allyliques ou (meth)acryliques portant des groupes fluorés ou perfluorés.

**8.** Composition selon l'une quelconque des revendications 2 à 7, **caractérisée par le fait que** le monomère

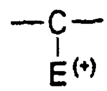

est choisi parmi les monomères (méth)acryliques, vinyliques, allyliques ou diallyliques portant une amine tertiaire E quatemisée par un halogénure d'alkyle ou un sulfate de dialkyle.

**9.** Composition selon l'une quelconque des revendications 2 à 8, **caractérisée par le fait que** le polymère de formule (I) est choisi dans le groupe constitué par les copolymères de styrène sulfonate de sodium/chlorure de trimethylammonio propyl (meth)acrylamide, les copolymères de styrène sulfonate de sodium/triméthyl ammonio éthylmethacrylate.

**10.** Composition selon la revendication 1, **caractérisée par le fait que** le polymère polyampholyte ait pour monomère de base celui qui répond à la formule suivante :

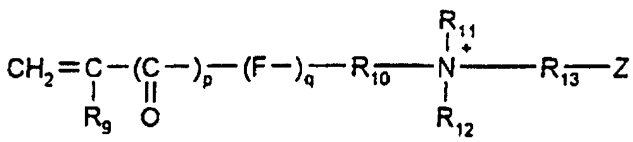 (II)

dans laquelle :

$R_9$, $R_{11}$ et $R_{12}$ désignent, identiques ou différents un hydrogène ou un alkyle en $C_1$-$C_4$, linéaire ou ramifie;
Z désigne $COO^{(-)}$, $SO_3^{(-)}$ ou $HPO_3^{(-)}$ ;
F désigne -NH ou O ou forme avec le groupe $R_{10}$ un cycle ou hétérocycle, aromatique ou non-aromatique en $C_5$-$C_7$;
$R_{10}$ et $R_{13}$ désignent, indépendamment l'un de l'autre, un groupe hydrocarboné divalent en particulier un groupe $-(CH_2)_n-$ avec n entier allant de 1 à 4 ;
$R_{10}$ peut former avec $R_{11}$ et $R_{12}$ un hétérocycle en $C_5$-$C_7$;
p vaut 0 ou 1 et q vaut 0 ou 1.

**11.** Composition selon la revendication 10, caractérisée parle fait que le polymère de formule (II) est choisi dans le groupe constitué par:

- le poly 1-vinyl-2-(3-sulfopropyl) imidazolium hydroxyde ;
- le poly 1-vinyl-3-(3-sulfopropyl) imidazolium hydroxyde ;
- le poly 1-vinyl-3-(4-sulfobutyl) imidazolium hydroxyde ;
- le poly 1-vinyl-2-méthyl-3-(4-sulfobutyl) imidazolium hydroxyde ;
- le poly 2-vinyl-1-(3-sulfopropyl) pyridinium hydroxyde ;
- le poly 2-méthyl-5-vinyl-1-(3-sulfopropyl) pyridinium hydroxyde ;
- le poly 4-vinyl-1-(3-sulfopropyl) pyridinium hydroxyde ;
- le poly diméthyl (2-méthacryloxyéthyl) (3-sulfopropyl) ammonium hydroxyde ;
- le polydiéthyl (2-méthacryloxyéthoxy-2-éthyl)(3-sulfopropyl) ammonium hydroxyde ;
- le poly 4-vinyl-4-(sulfobutyl) pyridinium hydroxyde ;
- le poly N-(3-sulfopropyl) N-méthacrylamidopropyl N,N-diméthyl ammonium bétaïne.

**12.** Composition selon l'une quelconque des revendications 1 à 11, **caractérisée par le fait que** le polymère polyampholyte est présent dans des concentrations allant de 0,01 à 20 % en poids et de préférence de 0,1 à 10 % en poids par rapport au poids total de la composition.

**13.** Compositon selon l'une quelconque des revendications 1 à 12, caractérisée parle fait que le polymère cationique a un poids moléculaire moyen en masse d'au moins 5.000 et plus préférentiellement d'au moins 10.000.

**14.** Composition selon la revendication 13, **caractérisée par le fait que** le poids moléculaire moyen en masse du polymère cationique varie de 10.000 à 10.000.000.

**15.** Composition selon l'une quelconque des revendications 1 à 14, **caractérisée par le fait que** la densité de charge cationique de polymère cationique varie de 0,9 à 4 meq/g et de préférence de 1,1 à 3 meq/g.

**16.** Composition selon l'une quelconque des revendications 1 à 15, **caractérisée par le fait que** le polymère cationique est choisi dans le groupe constitué par :

a) les copolymères de monomères vinyliques ayant des fonctions amines primaires, secondaires, tertiaires ou ammoniums quaternaires
b) les polysaccharides cationiques dérivés de l'amidon ou dérivés de la cellulose ;
c) les polymères cationiques dérivés de la gomme de guar ;
d) les éthers celluloses quaternaires, les copolymères de cellulose étherifiés et d'amidon.

**17.** Composition selon la revendication 16, **caractérisée par le fait que** les polysaccharides cationiques du paragraphe b) sont choisis parmi les polymères de formule:

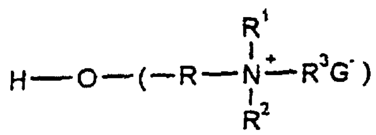

où:

H est un reste d'anhydroglucose tel que l'amidon ou un reste d'anhydroglucose cellulosique ;
R est un aikylène, un oxyalkylène, un polyoxyalkylène ou un hydroxyalkylène ou leurs melanges;
$R^1$, $R^2$ et $R^3$, identiques ou différents, désignent un groupe alkyle, aryle, alkylaryle, arylalkyle, alkoxyalkyle ou alkoxyaryle ; chaque groupe contenant jusqu'à 18 atomes de carbone et le nombre total d'atomes de carbone par unité cationique est de préférence inférieure ou égale à 20.
$G^-$ est un anion résultant de la quatemisation de l'amine $NR^1R^2R^3$.

**18.** Composition selon l'une quelconque des revendications 1 à 17, **caractérisée par le fait que** le polymère cationique est présent dans des proportions allant de 0,01 à 5 % en poids et de préférence de 0,1 à 3 % en poids par rapport au poids total de la composition.

**19.** Composition selon l'une quelconque des revendications 1 à 18, **caractérisée par le fait qu'**elle contient au moins un électrolyte minéral ou organique.

**20.** Composition selon la revendication 19, **caractérisée par le fait que** l'électrolyte est présent dans des concentrations allant de 0,1 à 30 % en poids et de préférence de 1 à 10 % en poids.

**21.** Composition selon l'une quelconque des revendications 1 à 20, **caractérisée par le fait que** le pH est ajusté entre 3 et 11.

**22.** Composition selon l'une quelconque des revendications 1 à 21, **caractérisée par le fait qu'**elle comporte en plus une base lavante constituée d'au moins un tensioactif ou d'un mélange de tensioactifs choisis dans le groupe des tensioactifs anioniques, cationiques, non-ioniques, amphotères ou zwittérioniques.

**23.** Composition selon la revendication 22, **caractérisée par le fait que** ia base lavante représente de 4 à 30 % en poids du poids total de la composition.

**24.** Composition selon l'une quelconque des revendications 1 à 23, **caractérisée par le fait que** le milieu cosmétiquement ou dermatologiquement acceptable est constituée d'eau ou d'un mélange d'eau et d'alcool inférieur.

**25.** Composition selon l'une quelconque des revendications 1 à 24, **caractérisée par le fait qu'**elle contient en plus des adjuvants choisi dans le groupe constitué par les parfums, les conservateurs, les sequestrants, les épaississants, les adoucissants, les modificateurs de mousse, les colorants, les nacrants, les hydratants, les antipelliculaires, les antiséborrhéiques, les vitamines, les filtres solaires.

**26.** Composition selon l'une quelconque des revendications 1 à 25, **caractérisée par le fait qu'**elle se présente sous la forme d'un liquide plus ou moins épaissi, d'une lotion, d'une crème ou d'un gel.

**27.** Composition selon l'une quelconque des revendications 1 à 26, **caractérisée par le fait qu'**elle est un produit à rincer pour le lavage, le soin et/ou le coiffage des cheveux.

**28.** Procédé de traitement non thérapeutique des cheveux, **caractérisé par le fait qu'**on applique directement sur les cheveux une composition selon l'une quelconque des revendications 1 à 27, que l'on effectue après un éventuel temps de pose, un rinçage à l'eau.

**Patentansprüche**

**1.** Zusammensetzung für die Behandlung von Keratinmaterialien, **dadurch gekennzeichnet, daß** sie in einem kosmetisch und/oder dermatologisch akzeptablen Medium mindestens enthält:

a) ein polyampholytisches Polymer, das aus mindestens einem ethylenisch ungesättigten Monomer besteht und das in der Kette oder seitlich an die Kette angebunden äquimolare oder nahezu äquimolare Mengen negativer Ladungen und positiver Ladungen aufweist, wobei das Polymer in Wasser bei 20 °C in einer Konzentration von 1 Gew.-% oder darüber unlöslich ist;
b) ein kationisches Polymer, dessen kationische Ladungsdichte kleiner als oder gleich 4 mÄq/g ist.

**2.** Zusammensetzung nach Anspruch 1, **dadurch gekennzeichnet, daß** das polyampholytische Polymer unter den Polymeren der folgenden Formel (1)

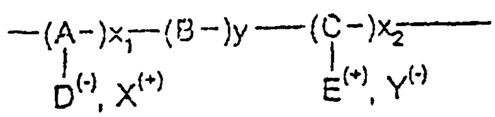

(I)

ausgewählt ist, in der bedeuten:

- A- eine Gruppe, die bei der Copolymerisation mindestens eines ethylenisch ungesättigten Monomers entsteht und die eine Gruppe $D^{(-)}$ trägt;
- $D^{(-)}$ eine anionische Gruppe, die ausgewählt ist unter

(i) $-COO^-$;
(ii) $-SO_3^-$;
(iii) $-PO_3^{2-}$;
(iv) $-HPO_3^-$;

- $X^{(+)}$ ein Kation, das bei der Neutralisation der Gruppen D mit einer anorganischen oder organischen Base gebildet wird;
- B- eine Gruppe, die bei der Copolymerisation mindestens eines hydrophilen oder hydrophoben, ethylenisch ungesättigten Monomers entsteht,
- C- eine Gruppe, die bei der Copolymerisation mindestens eines ethylenisch ungesättigten Monomers entsteht und die eine Gruppe $-E^{(+)}$ trägt;
- $E^{(+)}$ eine kationische Gruppe, die ausgewählt ist unter

(i)

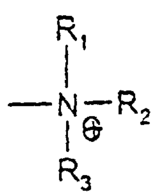

,

worin $R_1$, $R_2$ und $R_3$, gleich oder verschieden, Wasserstoff, geradkettiges, verzweigtes oder cyclisches (cycloaliphatisch oder aromatisch) $C_1$-$C_{22}$-Alkyl bedeuten;

(ii) 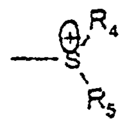 ,

worin $R_4$ und $R_5$, gleich oder verschieden, eine aliphatische, cycloaliphatische oder aromatische Gruppe bedeuten;

(iii) 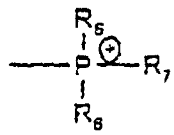 ,

worin $R_6$, $R_7$ und $R_8$, gleich oder verschieden, eine aliphatische, cycloaliphatische oder aromatische Gruppe bedeuten;
- $Y^{(-)}$ ein Anion, das bei der Neutralisation der Gruppen E mit einer anorganischen oder organischen Säure oder durch Quaternisierung der Gruppe E gebildet wird;
- $x_1$, $x_2$, y die in Mol angegebene Konzentration an Gruppen A, Gruppen B bzw. Gruppen C,

wobei $x_1$ und $x_2$ identisch oder nahezu identisch sind, damit die Gesamtladung des Polymers bei einem pH-Wert von etwa 7 etwa Null beträgt.

3. Zusammensetzung nach Anspruch 2, **dadurch gekennzeichnet, daß** in der Formel (I) die Summe $x_1 + x_2$ größer als oder gleich 40 Mol-% ist und y kleiner als oder gleich 60 Mol-% ist.

4. Zusammensetzung nach Anspruch 2 oder 3, **dadurch gekennzeichnet, daß** in der Formel (I) D(-) Carboxylat bedeutet und die Gruppe

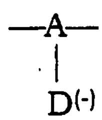

ausgewählt ist unter den Salzen geradkettiger, verzweigter oder cyclischer Carbonsäuren, den Salzen geradkettiger, verzweigter oder cyclischer Dicarbonsäuren und ihren Monoestern und Monoamiden.

5. Zusammensetzung nach Anspruch 2 oder 3, **dadurch gekennzeichnet, daß** in der Formel (I) $D^{(-)}$ Sulfonat bedeutet und die Gruppe

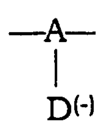

ausgewählt ist unter den Salzen der Aciylamido-2-methyl-2-propansulfonsäure, der Vinylsulfonsäure, der Styrolsulfonsäure, den Salzen des 2-Sulfoethyl(meth)acrylats.

6. Zusammensetzung nach Anspruch 2 oder 3, **dadurch gekennzeichnet, daß** D(-) Phosphonat bedeutet und

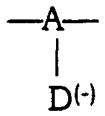

ein Salz der Vinylphosphonsäure bedeutet.

**7.** Zusammensetzung nach einem der Ansprüche 2 bis 6, **dadurch gekennzeichnet, daß** die Gruppe -B- aus mindestens einem Monomer, das unter den geradkettigen, verzweigten oder cyclischen $C_1$-$C_{24}$-Vinylestern, den Olefinen, Styrol und den substituierten Styrol-Derivaten, den geradkettigen, verzweigten oder cyclischen $C_1$-$C_{24}$-Estern oder $C_1$-$C_{24}$-Amiden der (Meth)acrylsäure ausgewählt ist und gegebenenfalls mindestens einem Monomer besteht, das unter siliconhaltigen Makromeren, die eine endständige Vinylgruppe aufweisen, Vinylmonomeren, Allylmonomeren und (Meth)acrylmonomeren, die fluorhaltige oder perfluorierte Gruppen tragen, ausgewählt ist.

**8.** Zusammensetzung nach einem der Ansprüche 2 bis 7, **dadurch gekennzeichnet, daß** das Monomer

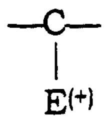

ausgewählt ist unter den (Meth)acryl-, Vinyl-, Allyl- und Diallylmonomeren, die ein tertiäres Amin E tragen, das mit einem Alkylhalogenid oder einem Dialkylsulfat quatemisiert ist.

**9.** Zusammensetzung nach einem der Ansprüche 2 bis 8, **dadurch gekennzeichnet, daß** das Polymer der Formel (I) unter den Natriumstyrolsulfonat/Trimethylammoniumpropyl(meth)acrylamidchlorid-Copolymeren und den Natriumstyrolsulfonat/Trimethylammoniumethylmethacrylat-Copolymeren ausgewählt ist.

**10.** Zusammensetzung nach Anspruch 1, **dadurch gekennzeichnet, daß** das polyampholytische Polymer als Grundmonomer das Monomer der folgenden Formel

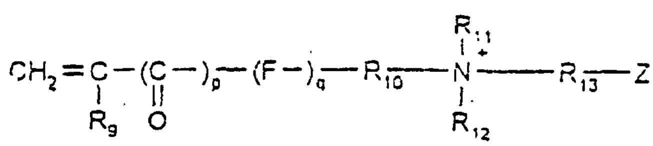

(II)

enthält, in der bedeuten:

$R_9$, $R_{11}$ und $R_{12}$, gleich oder verschieden, Wasserstoff oder geradkettiges oder verzweigtes $C_1$-$C_4$-Alkyl;
Z $COO^{(-)}$, $SO_3^{(-)}$ oder $HPO_3^{(-)}$;
F die Gruppe -NH oder O oder einen Rest, der zusammen mit der Gruppe $R_{10}$ einen aromatischen oder nichtaromatischen Ring oder Heterocyclus mit 5 bis 7 Kohlenstoffatomen bildet,
$R_{10}$ und $R_{13}$ unabhängig eine zweiwertige Kohlenwasserstoffgruppe, insbesondere eine Gruppe $-(CH_2)_n-$, worin n eine gänze Zahl im Bereich von 1 bis 4 bedeutet,
$R_{10}$ einen Rest, der mit $R_{11}$ und $R_{12}$ einen $C_5$-$C_7$-Heterocyclus bilden kann;
p die Zahl 0 oder 1 und q die Zahl 0 oder 1.

**11.** Zusammensetzung nach Anspruch 10, **dadurch gekennzeichnet, daß** das Polymer der Formel (II) ausgewählt ist unter:

- Poly(1-vinyl-2-(3-sulfopropyl)-imidazoliumhydroxid);
- Poly(1-vinyl-3-(3-sulfopropyl)-imidazoliumhydroxid);
- Poly(1-vinyl-3-(4-sulfobutyl)-imidazoliumhydroxid);

- Poly(1-vinyl-2-methyl-3-(4-sulfobutyl)-imidazoliumhydroxid);
- Poly(2-vinyl-1-(3-sulfopropyl)-pyridiniumhydroxid);
- Poly(2-methyl-5-vinyl-1-(3-sulfopropyl)-pyridiniumhydroxid);
- Poly(4-vinyl-1-(3-sulfopropyl)-pyridiniumhydroxid);
- Poly(dimethyl-(2-methacryloxyethyl)-(3-sulfopropyl)-ammoniumhydroxid);
- Poly(diethyl-(2-methacryloxyethoxy-2-ethyl)-(3-sulfopropyl)-ammoniumhydroxid);
- Poly(4-vinyl-4-(3-sulfobutyl)-pyridiniumhydroxid);
- Poly(N-(3-sulfopropyl)-N-methacrylamidopropyl-N,N-dimethylammoniumbetain.

**12.** Zusammensetzung nach einem der Ansprüche 1 bis 11, **dadurch gekennzeichnet, daß** das polyampholytische Polymer in einer Konzentration im Bereich von 0,01 bis 20 Gew.-% und vorzugsweise 0,1 bis 10 Gew.-%, bezogen auf das Gesamtgewicht der Zusammensetzung, enthalten ist.

**13.** Zusammensetzung nach einem der Ansprüche 1 bis 12, **dadurch gekennzeichnet, daß** das kationische Polymer ein Gewichtsmittel des Molekulargewichts von mindestens 5000 und bevorzugter von mindestens 10000 aufweist.

**14.** Zusammensetzung nach Anspruch 13, **dadurch gekennzeichnet, daß** das Gewichtsmittel des Molekulargewichts des kationischen Polymers im Bereich von 10000 bis 10000000 liegt.

**15.** Zusammensetzung nach einem der Ansprüche 1 bis 14, **dadurch gekennzeichnet, daß** die kationische Ladungsdichte des kationischen Polymers im Bereich von 0,9 bis 4 mÄq/g und vorzugsweise 1,1 bis 3 mÄq/g liegt.

**16.** Zusammensetzung nach einem der Ansprüche 1 bis 15, **dadurch gekennzeichnet, daß** das kationische Polymer ausgewählt ist unter

a) den Copolymeren von Vinylmonomeren, die primäre, sekundäre, tertiäre Amingruppen oder quartäre Ammoniumgruppen tragen,
b) den kationischen Polysacchariden, die von Stärke oder Cellulose abgeleitet sind,
c) den kationischen Polysacchariden, die von Guargummi abgeleitet sind,
d) den quartären Celluloseethern, den veretherten Copolymeren von Cellulose und Stärke.

**17.** Zusammensetzung nach Anspruch 16, **dadurch gekennzeichnet, daß** die kationischen Polysaccharide gemäß Abschnitt b) unter den Polymeren der Formel

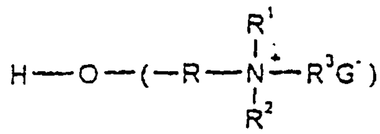

ausgewählt sind, in der bedeuten:

H einen Anhydroglucoserest, wie Stärke, oder einen Celluloseanhydroglucoserest
R Alkylen, Oxyalkylen, Polyoxyalkylen oder Hyroxyalkylen oder Gemische dieser Reste,
$R^1$, $R^2$ und $R^3$, gleich oder verschieden, Alkyl, Aryl, Alkylaryl, Arylalkyl, Alkoxyalkyl oder Alkoxyaryl, wobei jede dieser Gruppen bis zu 18 Kohlenstoffatome enthält, wobei die Gesamtzahl der Kohlenstoffatome pro kationischer Einheit vorzugsweise kleiner als oder gleich 20 ist,
$G^-$ ein Anion, das bei der Quaternisierung des Amins $NR^1R^2R^3$ entsteht.

**18.** Zusammensetzung nach einem der Ansprüche 1 bis 17, **dadurch gekennzeichnet, daß** das kationische Polymer in einem Anteil von 0,01 bis 5 Gew.-% und vorzugsweise 0,1 bis 3 Gew.-%, bezogen auf das Gesamtgewicht der Zusammensetzung, enthalten ist.

**19.** Zusammensetzung nach einem der Ansprüche 1 bis 18, **dadurch gekennzeichnet, daß** sie mindestens einen anorganischen oder organischen Elektrolyten enthält.

**20.** Zusammensetzung nach Anspruch 19, **dadurch gekennzeichnet, daß** der Elektrolyt in einer Konzentration von

0,1 bis 30 Gew.-% und vorzugsweise 1 bis 10 Gew.-% enthalten ist.

**21.** Zusammensetzung nach einem der Ansprüche 1 bis 20, **dadurch gekennzeichnet, daß** der pH-Wert auf einen Wert im Bereich von 3 bis 11 eingestellt ist.

**22.** Zusammensetzung nach einem der Ansprüche 1 bis 21, **dadurch gekennzeichnet, daß** sie außerdem eine reinigende Grundlage enthält, die aus mindestens einem grenzflächenaktiven Stoff oder einem Gemisch grenzflächenaktiver Stoffe besteht, die unter den anionischen, kationischen, nichtionischen, amphoteren und zwitterionischen grenzflächenaktiven Stoffen ausgewählt sind.

**23.** Zusammensetzung nach Anspruch 22, **dadurch gekennzeichnet, daß** die reinigende Grundlage 4 bis 30 Gew.-% des Gesamtgewichts der Zusammensetzung ausmacht.

**24.** Zusammensetzung nach einem der Ansprüche 1 bis 23, **dadurch gekennzeichnet, daß** das kosmetisch oder dermatologisch akzeptable Medium aus Wasser oder einem Gemisch aus Wasser und einem niederen Alkohol besteht.

**25.** Zusammensetzung nach einem der Ansprüche 1 bis 24, **dadurch gekennzeichnet, daß** sie außerdem Hilfsstoffe enthält, die ausgewählt sind unter Parfüms, Konservierungsmitteln, Maskierungsmitteln, Verdickungsmitteln, reizlindernden Mitteln, schaummodifizierenden Mitteln, Farbmitteln, Perlglanzpigmenten, Hydratisierungsmitteln, Antischuppenmitteln, Antiseborrhöika, Vitaminen, Sonnenschutzfiltern.

**26.** Zusammensetzung nach einem der Anspruche 1 bis 25, **dadurch gekennzeichnet, daß** sie in Form einer mehr oder weniger verdickten Flüssigkeit, einer Lotion, einer Creme oder eines Gels vorliegt.

**27.** Zusammensetzung nach einem der Ansprüche 1 bis 26, **dadurch gekennzeichnet, daß** es sich um ein auszuspülendes Produkt zum Waschen, Pflegen und/oder Frisieren der Haare handelt.

**28.** Verfahren zur nicht-therapeutischen Behandlung der Haare, **dadurch gekennzeichnet, daß** eine Zusammensetzung nach einem der Ansprüche 1 bis 27 unmittelbar auf die Haare aufgetragen wird und daß die Zusammensetzung nach einer möglichen Einwirkungszeit mit Wasser aus den Haaren ausgespült wird.

## Claims

**1.** Composition for treating keratin substances, **characterized in that** it contains, in a cosmetically and/or dermatologically acceptable aqueous medium, at least:

a) one polyampholytic polymer consisting of at least one monomer containing ethylenic unsaturation and containing, in the chain or attached laterally to the chain, equimolar or virtually equimolar amounts of negative charges and positive charges; the said polymer being insoluble in water at a concentration greater than or equal to 1% by weight and at 20°C;
b) one cationic polymer whose cationic charge density is less than or equal to 4 meq/g.

**2.** Composition according to Claim 1, **characterized in that** the polyampholytic polymer is chosen from polymers corresponding to the following formula:

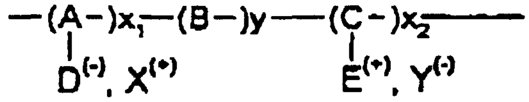

(I)

in which:

- A- denotes a group resulting from the copolymerization of at least one monomer containing ethylenic unsaturation and bearing a group $D^{(-)}$ ;
- $D^{(-)}$ denotes an anionic group chosen from the group consisting of:

(i) $-COO^{(-)}$ ;
(ii) $-SO_3^{(-)}$ ;
(iii) $-PO_3^{(2-)}$ ;
(iv) $-HPO_3^{(-)}$ ;

- X+ denotes a cation derived from the neutralization of the groups D by an inorganic or organic base;
- B- is a group resulting from the copolymerization of at least one hydrophobic or hydrophilic monomer containing ethylenic unsaturation;
- C- is a group resulting from the copolymerization of at least one monomer containing ethylenic unsaturation and bearing a group $-E^{(+)}$ ;
- $E^{(+)}$ denotes a cationic group chosen from the group consisting of:

(i) 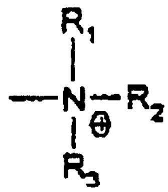

in which $R_1$, $R_2$ and $R_3$, which may be identical or different, denote hydrogen or a linear, branched or cyclic (cycloaliphatic or aromatic) $C_1$-$C_{22}$ alkyl group;

(ii) 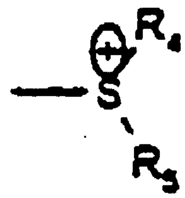

in which $R_4$ and $R_5$, which may be identical or different, denote an aliphatic, cycloaliphatic or aromatic group;

(iii) 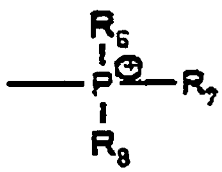

in which $R_6$, $R_7$ and $R_8$, which may be identical or different, denote an aliphatic, cycloaliphatic or aromatic group;
- $Y^{(-)}$ denotes an anion resulting from the neutralization of the groups E by an inorganic or organic acid or from the quaternization of the groups E;
$x_1$, $x_2$ and y respectively denote the molar concentration of group A, of group B and of group C;
$x_1$ and $x_2$ being identical or virtually identical, such that the overall charge of the polymer is close to 0 for a pH in the region of 7.

3. Composition according to Claim 2, **characterized in that**, in formula (I), the sum $x_1 + x_2$ is greater than or equal to 40 mol% and y is less than or equal to 60 mol%.

4. Composition according to Claim 2 or 3,
**characterized in that**, in formula (I), $D^{(-)}$ denotes carboxylate and

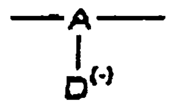

is chosen from the group consisting of linear, branched or cyclic carboxylic acid salts; salts of linear, branched or cyclic dicarboxylic acids and their monoesters or monoamides.

**5.** Composition according to Claim 2 or 3,
**characterized in that**, in formula (I), $D^{(-)}$ denotes suiphonate and

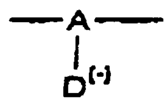

is chosen from the group consisting of salts of 2-acrylamido-2-methylpropanesulphonic acid, of vinylsulphonic acid, of styrenesulphonic acid and salts of 2-sulphoethyl methacrylate.

**6.** Composition according to Claim 2 or 3,
**characterized in that** $D^{(-)}$ denotes phosphonate and

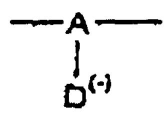

denotes a vinylphosphonic acid salt.

**7.** Composition according to any one of Claims 2 to 6, **characterized in that** the group -B- consists of at least one monomer chosen from linear, branched or cyclic $C_1$-$C_{24}$ vinyl esters, olefins, styrene and its substituted derivatives, linear, branched or cyclic $C_1$-$C_{24}$ esters or amides of (meth)acrylic acid, and optionally of at least one monomer chosen from the group consisting of silicone macromers having a terminal vinyl function, vinylic, allylic or (meth) acrylic monomers bearing fluoro or perfluoro groups.

**8.** Composition according to any one of Claims 2 to 7,
**characterized in that** the monomer

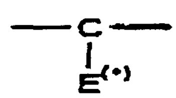

is chosen from (meth)acrylic, vinylic, allylic or diallylic monomers bearing a tertiary amine E quaternized with an alkyl halide or a dialkyl sulphate.

**9.** Composition according to any one of Claims 2 to 8, **characterized in that** the polymer of formula (I) is chosen from the group consisting of copolymers of sodium styrenesulphonate/trimethylammoniopropyl-(meth)acrylamide chloride and copolymers of sodium styrenesulphonate/trimethylammonioethyl methacrylate.

**10.** Composition according to Claim 1, **characterized in that** the polyampholytic polymer has as its base monomer one which corresponds to the following formula:

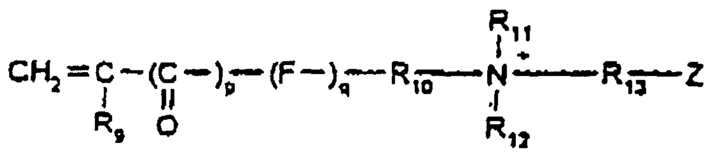 (II)

in which:

$R_9$, $R_{11}$ and $R_{12}$, which may be identical or different, denote a hydrogen or a linear or branched $C_1$-$C_4$ alkyl;
Z denotes $COO^{(-)}$, $SO_3^{(-)}$ or $HPO_3^{(-)}$;
F denotes -NH or O or forms, with the group $R_{10}$, an aromatic or non-aromatic $C_5$-$C_7$ ring or heterocycle;
$R_{10}$ and $R_{13}$ denote, independently of each other, a divalent hydrocarbon group, in particular a $-(CH_2)_{-n}-$ group with n being an integer ranging from 1 to 4;
$R_{10}$ can form, with $R_{11}$ and $R_{12}$, a $C_5$-$C_7$ heterocycle;
p is equal to 0 or 1 and q is equal to 0 or 1.

**11.** Composition according to Claim 10, **characterized in that** the polymer of formula (II) is chosen from the group consisting of:

- poly 1-vinyl-2-(3-sulphopropyl)imidazolium hydroxide;
- poly 1-vinyl-3-(3-sulphopropyl)imidazolium hydroxide;
- poly 1-vinyl-3-(4-sulphobutyl)imidazolium hydroxide;
- poly 1-vinyl-2-methyl-3-(4-sulphobutyl)imidazolium hydroxide;
- poly 2-vinyl-1-(3-sulphopropyl)pyridinium hydroxide;
- poly 2-methyl-5-vinyl-1-(3-aulphopropyl)pyridinium hydroxide;
- poly 4-vinyl-1-(3-sulphopropyl)pyridinium hydroxide;
- polydimethyl (2-methacryloxyethyl) (3-sulphopropyl) - ammonium hydroxide;
- polydiethyl(2-mathacryloxyethoxy-2-ethyl) (3-sulphopropyl)ammonium hydroxide;
- poly 4-vinyl-4-(sulphobutyl)pyridinium hydroxide;
- poly N-(3-sulphopropyl)-N-methacrylamidopropyl-N,N-dimethylammonium betaine.

**12.** Composition according to any one of Claims 1 to 11, **characterized in that** the polyampholytic polymer is present in concentrations ranging from 0.01 to 20% by weight, and preferably from 0.1 to 10% by weight, relative to the total weight of the composition.

**13.** Composition according to any one of Claims 1 to 12, **characterized in that** the cationic polymer has a weight-average molecular weight of at least 5 000 and more preferably of at least 10 000.

**14.** Composition according to Claim 13, **characterized in that** the weight-average molecular weight of the cationic polymer ranges from 10 000 to 10 000 000.

**15.** Composition according to any one of Claims 1 to 14, **characterized in that** the cationic charge density of the cationic polymer ranges from 0.9 to 4 meq/g and preferably from 1.1 to 3 meq/g.

**16.** Composition according to any one of Claims 1 to 15, **characterized in that** the cationic polymer is chosen from the group consisting of:

a) copolymers of vinylic monomers having primary, secondary or tertiary amine or quaternary ammonium functions;
b) cationic polysaccharides derived from Starch or derived from cellulose;
c) cationic polymers derived from guar gum;
d) quaternary cellulose ethers and etherified copolymers of cellulose and of starch.

**17.** Composition according to Claim 16, **characterized in that** the cationic polysaccharides of paragraph b) are chosen from the polymers of formula:

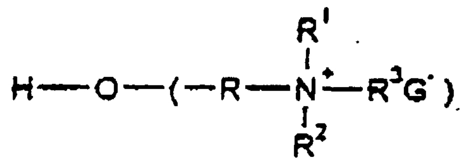

in which:

H is an anhydroglucose residue such as starch or a cellulosic anhydroglucose residue;
R is an alkylene, an oxyalkylene, a polyoxyalkylene or a hydroxyalkylene or mixtures thereof;
$R^1$, $R^2$ and $R^3$, which may be identical or different, denote an alkyl, aryl, alkylaryl, arylalkyl, alkoxyalkyl or alkoxyaryl group; each group containing up to 18 carbon atoms and the total number of carbon atoms per cationic unit is preferably less than or equal to 20;
$G^-$ is an anion resulting from the quaternization of the amine $NR^1R^2R^3$.

**18.** Composition according to any one of Claims 1 to 17, **characterized in that** the cationic polymer is present in proportions ranging from 0.01 to 5% by weight, and preferably from 0.1 to 3% by weight, relative to the total weight of the composition.

**19.** Composition according to any one of Claims 1 to 18, **characterized in that** it contains at least one inorganic or organic electrolyte.

**20.** Composition according to Claim 19, **characterized in that** the electrolyte is present in concentrations ranging from 0.1 to 30% by weight, and preferably from 1 to 10% by weight.

**21.** Composition according to any one of Claims 1 to 20, **characterized in that** the pH is adjusted to between 3 and 11.

**22.** Composition according to any one of Claims 1 to 21, **characterized in that** it also contains a washing base consisting of at least one surfactant or of a mixture of surfactants chosen from the group of anionic, cationic, nonionic, amphoteric and zwitterionic surfactants.

**23.** Composition according to Claim 22, **characterized in that** the washing base represents from 4 to 30% by weight of the total weight of the composition.

**24.** Composition according to any one of Claims 1 to 23, **characterized in that** the cosmetically or dermatologically acceptable medium consists of water or a mixture of water and a lower alcohol.

**25.** Composition according to any one of Claims 1 to 24, **characterized in that** it also contains adjuvants chosen from the group consisting of fragrances, preserving agents, sequestering agents, thickeners, softeners, foam modifiers, dyes, pearlescent agents, moisturizers, antidandruff agents, anti-seborrhoeic agents, vitamins and sunscreens.

**26.** Composition according to any one of Claims 1 to 25, **characterized in that** it is in the form of a relatively thickened liquid, a lotion, a cream or a gel.

**27.** Composition according to any one of Claims 1 to 26, **characterized in that** it is a rinse-out product for washing, caring for and/or styling the hair.

**28.** Non-therapeutic treatment process for the hair, **characterized in that** a composition according to any one of Claims 1 to 27 is applied directly to the hair and, after optionally leaving it on the hair for a period of time, the hair is rinsed with water.